# EUROPÄISCHE PATENTSCHRIFT

(11) **EP 2 332 900 B1**
(45) Veröffentlichungstag und Bekanntmachung des Hinweises auf die Patenterteilung: **25.02.2015**
(21) Anmeldenummer: 10011677.1
(22) Anmeldetag: 04.05.2004
(51) Int. Cl.: C07C 51/235

(54) **Verfahren zur selektiven Kohlenhydrat-Oxidation unter Verwendung geträgerter Gold-Katalysatoren**
Method for selective carbohydrate oxidation using supported gold catalysts
Procédé d'oxydation sélective de glucides à l'aide de catalyseurs or supportés

(30) Priorität: 05.05.2003 DE 10319917
(43) Veröffentlichungstag der Anmeldung: 15.06.2011
(62) Teilanmeldung aus: 04730988.5
(73) Patentinhaber: Südzucker AG Mannheim/Ochsenfurt, 68165 Mannheim (DE)
(72) Erfinder: Kowalczyk, Jörg, 67304 Eisenberg/Steinborn (DE); Begli, Alireza Haji, 67305 Ramsen (DE); Prüsse, Ulf, 38118 Braunschweig (DE); Berndt, Heinz, 12623 Berlin (DE); Pitsch, Irene, 12487 Berlin (DE)
(74) Vertreter: Schwahn, Hartmut

(56) Entgegenhaltungen:
- DE-A1- 3 535 720
- US-A- 4 985 553
- BIELLA S ET AL: "Selective Oxidation of D-Glucose on Gold Catalyst", JOURNAL OF CATALYSIS, ACADEMIC PRESS, DULUTH, MN, US, Bd. 206, Nr. 2, 10. März 2002 (2002-03-10) , Seiten 242-247, XP004445070, ISSN: 0021-9517, DOI: DOI:10.1006/JCAT.2001.3497
- K. HEYNS ET AL.: "Konfigurations- und Konformationsselektivität bei katalystischen Oxydationen mit Sauerstoff am Platin-Kontakt", FORTSCHRITTE DER CHEMISCHEN FORSCHUNG, vol. 11, 1969, pages 350-353,
- H. Van Bekkum: "Studies on Selective Carbohydrate Oxidation", 1991, VCH pages 289-310, * the whole document *
- M. KUNZ ET AL.: "Katalytische Oxidation von Isomaltulose", CHEM. ING. TECH., vol. 67, no. 7, 1995, pages 836-842,

## Beschreibung

Die vorliegende Erfindung betrifft ein Verfahren zur selektiven Oxidation eines Kohlenhydrates in Gegenwart eines Gold-Katalysators, der nanodispers verteilte Gold-Partikel auf einem Kohlenstoff- Träger umfasst, sowie die unter Verwendung dieser Verfahren hergestellten Aldonsäure-Oxidationsprodukte.

Bei der Käse-Produktion und der Molkeverarbeitenden Industrie fallen jährlich weltweit etwa 1,2 Mio. Tonnen Lactose als Abfallprodukt an. Lactose als wichtigster Kohlenhydrat-Bestandteil der Milch hat bislang jedoch kaum wirtschaftliche Bedeutung. Einer der Gründe dafür ist in der Lactose-Intoleranz von Teilen der Bevölkerung zu suchen. Personen mit Lactose-Intoleranz können Lactose nicht verwerten und reagieren bei Lactose-Konsum mit Unverträglichkeitssymptomen wie Durchfall. Nur ein relativ geringer Teil der anfallenden Lactose wird einer wirtschaftlichen Verwertung zugeführt, wobei Lactose beispielsweise als Fermentationssubstrat, als Füllmittel oder zur Herstellung von Diät-Lebensmitteln eingesetzt wird. Der größte Teil der anfallenden Lactose-Mengen wird jedoch über die Abwasseranlagen der Produzenten entsorgt, was zu Störungen des ökologischen Gleichgewichtes in Gewässern führen kann. Da Lactose jedoch ein in großen Mengen vorhandener und äußerst kostengünstiger Rohstoff ist, besteht großes Interesse an einer wirtschaftlichen Aufwertung dieses Kohlenhydrates. In jüngster Zeit wurden daher insbesondere verschiedene enzymatische Spaltungs- und Transformationsreaktionen entwickelt, bei denen Lactose als Ausgangsmaterial zur Herstellung höherwertige Produkte eingesetzt wird.

Durch Oxidation von Lactose kann so Lactobionsäure erhalten werden, welche für eine Reihe von Anwendungen äußerst interessant ist. Bislang wird zur Lactobionsäure-Herstellung aus Lactose ein enzymatisches Verfahren unter Verwendung der Enzyme Cellobiose-Dehydrogenase und Hexose-Oxidase eingesetzt. Der nicht zufriedenstellende Umsatz beispielsweise bei der Cellobiose-Dehydrogenasekatalysierten Reaktion lässt sich unter Verwendung des Enzyms Laccase steigern, das die bei der Reaktion reduzierten Redox-Mediatoren reoxidiert. Aufgrund ihrer exzellenten Fähigkeit, Metallchelate zu bilden, wird Lactobionsäure unter anderem in der sogenannten "Wisconsin-Transplantations-Lösung" eingesetzt, da Lactobionsäure den durch Metallionen bedingten oxidativen Schaden während der Lagerung von zu transplantierenden Organen reduzieren kann. Lactobionsäure lässt sich ebenfalls als biologisch abbaubarer Cobuilder in Waschpulver einsetzen, das bis zu 40 % Lactobionsäure enthalten kann. Aufgrund des milden süßlich-sauren Geschmacks von Lactobionsäure bestehen weitere Anwendungsmöglichkeiten in der Lebensmitteltechnologie.

Auch für andere Aldonsäuren beziehungsweise Oligosaccharid-Aldonsäuren besteht ein großes Einsatzpotential in der pharmazeutischen Industrie, der Kosmetik-Herstellung und in der Lebensmitteltechnologie. Aldonsäuren werden derzeit hauptsächlich mittels mikrobieller oder enzymatischer Umwandlung aus den entsprechenden Mono- oder Oligosacchariden hergestellt. So kann beispielsweise Glucose unter Verwendung von Acetobacter methanolicus in Gluconsäure umgewandelt werden. Die enzymatische Herstellung von Aldonsäuren ist jedoch generell durch eine relativ geringe Produktivität gekennzeichnet und auch aus Gründen des Umweltschutzes nicht unproblematisch. Daher besteht ein großes Interesse an alternativen Oxidationsverfahren, die zu einer deutlich geringeren Belastung der Umwelt führen, wobei das zu oxidierende Kohlenhydrat, beispielsweise ein Monosaccharid, unter Verwendung eines heterogenen Katalysators zu der entsprechenden Aldonsäure oxidiert wird.

Die heterogene Katalyse einer Oxidationsreaktion erfolgt üblicherweise in einem Drei-Phasen-Reaktor, wobei der feste, meist pulverförmige Katalysator in einer die zu oxidierende Verbindung enthaltenden Flüssigphase suspendiert ist und während der Reaktion Sauerstoff durch die Flüssigphase hindurchgeperlt wird. Obwohl die katalytische Oxidation im Vergleich zur enzymatischen Umsetzung einige erhebliche Vorteile bietet, insbesondere unter dem Gesichtspunkt der erheblich geringeren Umweltverschmutzung, weist sie jedoch einen entscheidenden Nachteil auf. Bei Verwendung von Metallen kann die Disauerstoff-Aktivierung zu radikalischen Reaktionen führen, die insbesondere im Fall polyfunktionaler Moleküle die Selektivität der Umsetzung deutlich senken können (Sheldon und Kochi, "Metal Catalyzed Oxidations of Organic Compounds", 1981, Academic Press, New York).

Bislang am besten untersucht ist die Verwendung geträgerter Palladium- und Platin-Katalysatoren zur Oxidation von Glucose. Dabei hat sich herausgestellt, dass bei Verwendung dieser Katalysatoren die katalytische Umsetzung von Glucose zu Gluconsäure aufgrund der geringen Selektivität und Umsetzungsrate erheblich eingeschränkt ist. Auch kommt es zu einer relativ schnellen Deaktivierung beider Katalysator-Typen. Diese Deaktivierung beruht offensichtlich entweder auf einer Blockierung der Katalysator-Oberfläche aufgrund der Adsorption von Molekülen oder auf Disauerstoff-bedingten Vergiftungseffekten (Van Dam, Kieboom und van Bekkum, Appl. Catal., 33 (1990), 187). Einige der Faktoren, die die katalytische Umsetzung von Glucose zu Gluconsäure einschränken, lassen sich durch die Einführung von Promotoren wie Bismut oder Blei deutlich verbessern. Neben einer Verbesserung der Katalysator-Lebensdauer erhöhen sich dadurch insbesondere die Selektivität und die Umsetzungsrate der Reaktion (Fiege und Wedemeyer, Angew. Chem., 93 (1981), 812; Wenkin et al., Appl. Catal. A: General, 148 (1996), 181).

Pd und Bi werden aufgrund eines möglichen Leachings dieser toxikologisch bedenklichen Substanzen jedoch kontrovers diskutiert. Zur Erhöhung der Umsetzungsgeschwindigkeit und zur Vermeidung der Katalysator-Deaktivierung sind leicht alkalische Bedingungen erforderlich. Unter solchen Bedingungen treten jedoch Nebenreaktionen auf, die die Gluconat-Produktion vermindern. Ebenfalls nachteilig ist, dass bei Verwendung von Basen anstelle der freien Gluconsäure Gluconat erzeugt wird (Biella, Prati und Rossi, Journal of Catalysis, 206 (2002), 242-247) .

Zur großtechnischen Produktion von Gluconsäure wird daher nach wie vor dem Fermentationsprozess der Vorrang gegeben, trotz der bei diesem Verfahren auftretenden Probleme, beispielsweise der starken Abwasser-Verunreinigung und der nicht unerheblichen Bildung von Nebenprodukten. Aus diesem Grund ist es erforderlich, neue Katalysator-Typen zu entwickeln, die eine katalytische Oxidation von Kohlenhydraten zur Herstellung von Aldonsäuren unter Verwendung von Disauerstoff als Oxidationsmittel ermöglichen und neben hoher Aktivität und Selektivität eine lange Lebensdauer aufweisen.

Bislang wurden geträgerte Gold-Katalysatoren vor allem zur Oxidation von CO oder Propen in der Gasphase und für Selektivhydrierungen eingesetzt. Biella et al., Journal of Catalysis, 206 (2002) 242-247, beschreiben die Verwendung eines Kohlenstoff-geträgerten Gold-Katalysators zur selektiven Oxidation von D-Glucose zu D-Gluconsäure in der Flüssigphase. Ein Vergleich zwischen dem Kohlenstoff-geträgerten Gold-Katalysator und herkömmlichen Palladium- und Platin-Katalysatoren zeigt, dass der Gold-Katalysator sowohl Palladium- als auch Platin-Katalysatoren in mehrfacher Hinsicht überlegen ist. Im Vergleich zu Palladium- und Platin-Katalysatoren erweist sich der verwendete Gold-Katalysator insbesondere wesentlich stabiler gegenüber einer Deaktivierung. Ein weiterer Vorteil des verwendeten Gold-Katalysators besteht darin, dass dieser bei der Glucose-Umsetzung keine externe pH-Kontrolle erfordert. Die bekannten Kohlenstoff-geträgerten Gold-Katalysatoren weisen jedoch einen erheblichen Nachteil auf. Einerseits wird mit abnehmendem pH-Wert vermehrt Gold aus dem Katalysator ausgelaugt. Andererseits wird mit steigendem pH-Wert das Wachstum der Goldpartikel gefördert. Beides führt zu einer Abnahme der KatalysatorAktivität. Das mit steigendem pH-Wert zunehmende Auflösen von Goldpartikeln geht mit einer Vergrößerung der Goldpartikel einher. Dies ist wahrscheinlich darauf zurückzuführen, dass kleine Goldpartikel in Lösung gehen und sich dann das Gold an größeren Gold-Partikeln abscheidet, wobei es zu einer Reduktion von Au(I,III)-Partikeln kommt.

Das der vorliegenden Erfindung zugrunde liegende technische Problem besteht darin, gegenüber den im Stand der Technik bekannten Katalysatoren verbesserte Gold-Katalysatoren bereitzustellen, die eine hohe Selektivität und Aktivität bezüglich der Oxidation von Kohlenhydraten zu den entsprechenden Aldonsäuren aufweisen und bei denen die im Stand der Technik bekannten Probleme von Gold-Katalysatoren, insbesondere der durch mehrfache Verwendung bedingte Aktivitätsabfall, dessen Ausmaß vom pH-Wert abhängig ist, nicht auftreten, so dass die Gold-Katalysatoren für die großtechnische Aldonsäure-Herstellung aus geeigneten Kohlenhydrat-Ausgangsmaterialien eingesetzt werden können. Darüber hinaus sollen die verbesserten Gold-Katalysatoren für die Oxidation einer Vielzahl unterschiedlicher Kohlenhydrate zu den entsprechenden Aldonsäuren eingesetzt werden können.

Das der vorliegenden Erfindung zugrunde liegende technische Problem wird durch ein Verfahren zur selektiven Oxidation von mindestens einem Oligosaccharid, einem Gemisch davon oder einer diese(s) enthaltenden Zusammensetzung gelöst, wobei eine wässrige Lösung des Oligosaccharides, des Gemisches oder der Zusammensetzung in Gegenwart eines Gold-Katalysators, umfassend nanodispers verteilte Gold-Partikel auf einem Träger, und von Sauerstoff umgesetzt wird.

Erfindungsgemäß ist vorgesehen, dass zur selektiven Oxidation von Oligosacchariden ein Gold-Katalysator eingesetzt wird, der nanodispers verteilte Gold-Partikel auf einem Träger umfasst, wobei der Träger ein Kohlenstoff-Träger ist.

Gemäß der Erfindung wird also zur selektiven Oxidation eines Oligosaccharides, eines Oligosaccharid-Gemisches oder einer diese(s) enthaltenden Zusammensetzung ein Kohlenstoff-geträgerter Gold-Katalysator eingesetzt.

Im Zusammenhang mit der vorliegenden Erfindung wird unter einem "Katalysator" ein Stoff verstanden, der die zum Ablauf einer bestimmten Reaktion benötigte Aktivierungsenergie herabsetzen und dadurch die Reaktionsgeschwindigkeit erhöhen kann, ohne im Endprodukt der Reaktion zu erscheinen. Unter einem "Gold-Katalysator" wird ein Katalysator verstanden, der Gold auf einem Trägermaterial umfasst oder aus Gold auf einem Trägermaterial besteht, wobei das Gold in Form von dispers verteilten Nanopartikeln auf dem Träger vorliegt. Die Gold-Nanopartikel weisen einen Durchmesser von weniger als 20 nm, vorzugsweise < 10 nm und besonders bevorzugt < 5 nm auf.

Unter einem "Kohlenstoff-Träger" wird im Zusammenhang mit der vorliegenden Erfindung insbesondere ein Aktivkohle-Träger verstanden. Die erfindungsgemäß eingesetzten Kohlenstoff-geträgerte Gold-Katalysatoren werden vorzugsweise unter Verwendung des Incipient-Wetness-Verfahrens oder über Gold-Sole hergestellt. Geeignete Verfahren zur Herstellung Kohlenstoff-geträgerter Gold-Katalysatoren, umfassen insbesondere geeignete Tränkungstechniken und Goldkolloid-Trägerung, beispielsweise unter Verwendung spezifischer Polymeren. Geeignete Verfahren zur Herstellung Kohlenstoff-geträgerter Gold-Katalysatoren sind beispielsweise in Prati und Martra, Gold Bulletin, 32(3) (1999), 96-101, beschrieben, wobei der Offenbarungsgehalt dieser Druckschrift durch Bezugnahme vollständig in den Offenbarungsgehalt der vorliegenden Erfindung einbezogen wird. Die erfindungsgemäß eingesetzten Kohlenstoff-geträgerten Gold-Katalysatoren weisen insbesondere Gold-Nanopartikel mit einem Durchmesser von weniger als 20 nm, vorzugsweise < 10 nm, besonders bevorzugt < 6 nm und am bevorzugtesten < 2 nm auf. Erfindungsgemäß enthalten die Kohlenstoff-geträgerten Gold-Katalysatoren etwa 0,1 % bis 5 %, Gold, vorzugsweise etwa 0,5 % bis 1,0 % Gold, besonders bevorzugt 0,5 % Gold.

Erfindungsgemäß wird der zur selektiven Oligosaccharid-Oxidation verwendete Kohlenstoff- geträgerte Gold-Katalysator in Flüssigphase eingesetzt, wobei der Katalysator als Feststoff vorliegt, während die Oligosaccharide in Flüssigphase vorhanden sind. Der zur Oxidation eingesetzte Disauerstoff wird als Gas durch die Flüssigphase hindurchgeperlt und durch intensives Rühren in der Flüssigphase gelöst. Der Kohlenstoff- geträgerte Gold-Katalysator wird vorzugsweise in Form eines Pulvers oder Granulats eingesetzt.

Unter "Oligosacchariden" werden im Zusammenhang mit der vorliegenden Erfindung Verbindungen verstanden, die durch Vereinigung von 2 bis 10 Monosaccharid-Molekülen unter Wasseraustritt erhalten werden und bei denen es sich um Glykoside oder Ether handelt. Der Begriff "Oligosaccharid" umfasst auch Derivate, also Abkömmlinge eines Oligosaccharides, die aus einem Oligosaccharid in ein oder mehreren Reaktionsschritten gebildet werden. Erfindungsgemäß bevorzugt handelt es sich bei den zu oxidierenden Oligosacchariden insbesondere um Disaccharide, Trisaccharide etc.. Die zu oxidierenden Oligosaccharide enthalten an ihrem C1-Kohlenstoffatom eine oxidierbare Aldehyd-Gruppe, die durch die Oxidation in eine Carboxy-Gruppe überführt wird. Hingegen werden Alkohol-Gruppen der Oligosaccharide nicht oxidiert. Bei den erfindungsgemäß eingesetzten Oligosacchariden kann es sich um Oligosaccharid-Aldosen handeln, die am C1-Kohlenstoff eine oxidierbare Aldehyd-Gruppe aufweisen, oder um Oligosaccharide in 2-Ketose-Form, bei denen am C1-Kohlenstoffatom eine oxidierbare Aldehyd-Funktion eingeführt werden kann.

Im Zusammenhang mit der vorliegenden Erfindung wird unter einer "Oligosaccharid-Aldose" ein Oligosaccharid verstanden, das am C1-Kohlenstoff eine Aldehyd-Gruppe (-CHO) besitzt. Durch die selektive Oxidation der Aldehyd-Gruppe einer Oligosaccharid-Aldose wird eine Oligosaccharid-Aldonsäure erhalten. Unter einer "Oligosaccharid-Aldonsäure" wird eine Oligosaccharidsäure verstanden, die durch Oxidation einer Aldehyd-Gruppe eines Oligosaccharides zur Carboxy-Gruppe erhalten wird.

Die vorliegende Erfindung betrifft daher ein Verfahren zur Herstellung einer Oligosaccharid-Aldonsäure oder eines Gemisches verschiedener Oligosaccharid-Aldonsäuren durch selektive Oxidation einer oder mehrerer Oligosaccharid-Aldosen mit einer oxidierbaren Aldehyd-Gruppe unter Verwendung eines Kohlenstoff- geträgerten Gold-Katalysators.

Unter einer "2-Ketose" wird ein Oligosaccharid verstanden, dass eine in 2-Stellung befindliche Keto-Gruppe besitzt. In 2-Ketose-Form vorliegende Oligosaccharide können in Oligosaccharid-Aldosen überführt werden, wobei die 2-Ketose zunächst in die Enol-Form überführt wird, die dann zu der oxidierbaren Oligosaccharid-Aldose tautomerisiert wird. Die vorliegende Erfindung betrifft daher auch ein Verfahren zur Herstellung einer Oligosaccharid-Aldonsäure oder eines Gemisches verschiedener Oligosaccharid-Aldonsäuren unter Verwendung einer oder mehrerer Oligosaccharide in 2-Ketose-Form, wobei die 2-Ketose(n) zunächst in die tautomere(n) Oligosaccharid-Aldose-Form(en) mit einer oxidierbaren Aldehyd-Gruppe überführt werden, die dann unter Verwendung eines Kohlenstoff- geträgerten Gold-Katalysators selektiv oxidiert wird/werden.

Im Zusammenhang mit der vorliegenden Erfindung wird unter einem "Oligosaccharid-Gemisch" ein Gemisch aus zwei oder mehr chemisch verschiedenen Oligosacchariden verstanden. Das zu oxidierende Oligosaccharid-Gemisch enthält in bevorzugter Ausführungs außer Oligosacchariden keine anderen Bestandteile. Bei dem zu oxidierenden Oligosaccharid-Gemisch kann es sich sowohl um ein homogenes Gemisch als auch ein heterogenes Gemisch handeln. Liegt das zu oxidierende Oligosaccharid-Gemisch nicht in flüssiger Form vor beziehungsweise enthält das zu oxidierende Oligosaccharid-Gemisch eine feste Phase, so wird das Gemisch vor der selektiven Oxidation in die Flüssigphase überführt, beispielsweise durch Herstellen einer wässrigen Lösung.

Eine Ausführungsform der Erfindung betrifft die Verwendung eines zu oxidierenden Oligosaccharid-Gemisches als Ausgangssubstrat, bei dem mindestens ein Oligosaccharid-Bestandteil ein C1-Kohlenstoffatom mit einer oxidierbaren Aldehyd-Gruppe enthält beziehungsweise bei dem vor der selektiven Oxidation das C1-Kohlenstoffatom mindestens eines Oligosaccharid-Bestandteils mit einer oxidierbaren Aldehyd-Funktion versehen werden kann.

Eine weitere bevorzugte Ausführungsform der Erfindung betrifft die Verwendung eines zu oxidierenden Oligosaccharid-Gemisches als Ausgangssubstrat, wobei das zu oxidierende Gemisch vorzugsweise mehrere Oligosaccharide enthält, die entweder an ihrem C1-Kohlenstoff bereits eine oxidierbare Aldehyd-Gruppe aufweisen oder an deren C1-Kohlenstoffatom vor der selektiven Oxidation eine oxidierbare Aldehyd-Gruppe eingeführt werden kann. In dieser Ausführungsform kann das zu oxidierende Oligosaccharid also mehrere unterschiedliche Oligosaccharid-Aldosen und/oder Oligosaccharide in 2-Ketose-Form umfassen. Besonders bevorzugt besteht das zu oxidierende Oligosaccharid-Gemisch ausschließlich aus Oligosaccharid-Einzelkomponenten, die entweder an ihrem C1-Kohlenstoff bereits eine oxidierbare Aldehyd-Gruppe aufweisen oder an deren C1-Kohlenstoffatom vor der selektiven Oxidation eine oxidierbare Aldehyd-Gruppe eingeführt werden kann. In dieser Ausführungsform besteht das zu oxidierende Oligosaccharid also ausschließlich aus unterschiedlichen Oligosaccharid-Aldosen und/oder Oligosacchariden in 2-Ketose-Form.

Im Zusammenhang mit der vorliegenden Erfindung wird unter einer "mindestens ein Oligosaccharid oder ein Oligosaccharid-Gemisch enthaltenden Zusammensetzung" ein Gemisch unterschiedlicher chemischer Verbindungen verstanden, wobei mindestens ein Bestandteil der Zusammensetzung ein Oligosaccharid ist, dessen C1-Kohlenstoffatom eine freie Aldehyd-Gruppe aufweist beziehungsweise vor der selektiven Oxidation mit einer oxidierbaren Aldehyd-Gruppe versehen werden kann. Bei den anderen Bestandteilen der zu oxidierenden Zusammensetzung kann es sich beispielsweise um andere Kohlenhydrate, beispielsweise nicht-oxidierbare oder selektiv oxidierbare Monosaccharide, Eiweißstoffe, Pektine, Säuren, Fette, Salze, Aromastoffe etc. handeln. Die zu oxidierende Zusammensetzung kann selbstverständlich mehrere unterschiedliche Oligosaccharid-Aldosen und/oder Oligosaccharide in 2-Ketose-Form enthalten.

In einer besonders bevorzugten Ausführungsform handelt es sich bei dem zu oxidierenden Oligosaccharid um ein Disaccharid. Das zu oxidierende Disaccharid kann eine Disaccharid-Aldose wie Maltose, Lactose, Cellobiose oder Isomaltose sein. Erfindungsgemäß wird bei der selektiven Oxidation von Maltose unter Verwendung des erfindungsgemäßen Verfahrens Maltobionsäure als Oxidationsprodukt erhalten. Unter Verwendung des erfindungsgemäßen Verfahrens wird bei der Lactose-Oxidation Lactobionsäure als Oxidationsprodukt erhalten.

In einer weiteren besonders bevorzugten Ausführungsform der Erfindung wird als zu oxidierendes Oligosaccharid eine Disaccharid-Ketose eingesetzt. Bei der zu oxidierenden Disaccharid-Ketose handelt es sich vorzugsweise um Palatinose. Vor der Oxidation wird Palatinose erfindungsgemäß in die tautomere Aldose-Form überführt, die dann oxidiert wird.

In einer weiteren besonders bevorzugten Ausführungsform der Erfindung handelt es sich bei dem zu oxidierenden Oligosaccharid-Gemisch beziehungsweise der zu oxidierenden Oligosaccharid-haltigen Zusammensetzung um ein Maltodextrin. Bei der selektiven Oxidation von Maltodextrin wird unter Verwendung des erfindungsgemäßen Verfahrens ein Oxidationsprodukt erhalten, das erfindungsgemäß einen Anteil von Maltobionsäure und Gluconsäure aufweist.

In einer weiteren besonders bevorzugten Ausführungsform ist die zu oxidierende Oligosaccharidhaltige Zusammensetzung ein Stärkesirup.

In bevorzugter Ausführungsform des erfindungsgemäßen Verfahrens zur selektiven Kohlenhydrat-Oxidation wird eine wässrige Lösung des zu oxidierenden Oligosaccharides, Oligosaccharid-Gemisches oder einer diese(s) enthaltenden Zusammensetzung hergestellt, die das Oligosaccharid oder das Oligosaccharid-Gemisch in einer Menge von mindestens etwa 10 mmol/l, bevorzugter mindestens etwa 100 mmol/l, 150 mmol/l, 200 mmol/l oder 250 mmol/l und am bevorzugtesten mindestens etwa 1000 mmol/l oder 1500 mmol/l enthält. Anschließend wird der wässrigen Oligosaccharid-Lösung der vorzugsweise pulverförmige Kohlenstoff- oder Metalloxid-geträgerte Gold-Katalysator in einer Menge von etwa 100 mg/l bis 10 g/l zugesetzt, wobei vorzugsweise pro Liter etwa 1 g Katalysator eingesetzt werden. Erfindungsgemäß beträgt das Verhältnis zwischen der Menge des/der zu oxidierenden Oligosaccharide(s) oder des Oligosaccharid-Gemisches und der Menge des auf dem Metalloxid-Trägers oder Kohlenstoff-Trägers enthaltenen Goldes mindestens etwa 300, bevorzugter mehr als 350, 500, 1000, 1500, 2000, 2500, 3000, 3500 oder 4000 und am bevorzugtesten mehr als 9000, 10 000, 15 000, 20 000, 25 000, 30 000, 35 000 oder 40 000.

In bevorzugter Ausführungsform des erfindungsgemäßen Verfahrens zur selektiven Oligosaccharid-Oxidation wird die selektive Oxidation des mindestens einen Oligosaccharides, des Oligosaccharid-Gemisches oder der diese(s) enthaltenden Zusammensetzung bei einem pH-Wert von 7 bis 11, vorzugsweise 8 bis 10, durchgeführt. Zur Durchführung der Kohlenhydrat-Oxidation wird erfindungsgemäß eine Temperatur von 20°C bis etwa 140°C, vorzugsweise 40°C bis 90°C, besonders bevorzugt 50°C bis 80°C eingesetzt. Erfindungsgemäß beträgt der Druck etwa 1 bar bis etwa 25 bar. Während der Oxidation wird erfindungsgemäß Sauerstoff und/oder Luft mit einer Begasungsrate von 100 ml/ (min x L_{Reaktorvolumen}) bis 10000 ml/ (min x L_{Reaktorvolumen}) vorzugsweise von 500 ml/ (min x L_{Reaktorvolumen}) , durch die wässrige Lösung des Oligosaccharides, des Gemisches oder der Zusammensetzung hindurchgeperlt.

Es können unter Verwendung der erfindungsgemäßen Verfahren Oxidationsprodukte hergestellt werden, die durch selektive Oxidation von mindestens einem Oligosaccharid, einem Oligosaccharid-Gemisch oder eine diese(s) enthaltende Zusammensetzung unter Verwendung eines Kohlenstoff- geträgerten Gold-Katalysators erhalten werden. Je nach verwendetem Ausgangsmaterial kann es sich bei den erfindungsgemäß hergestellten Oxidationsprodukten entweder um eine Aldonsäure, ein Gemisch unterschiedlicher Aldonsäuren oder eine Zusammensetzung handeln, die neben Bestandteilen wie Fetten, Pektinen, Eiweißstoffen, Salzen, Aromastoffen etc. ein oder mehrere Aldonsäuren umfasst. Insbesondere wenn als Edukt ein einziges Kohlenhydrat eingesetzt wird, weisen die erfindungsgemäß erhaltenen Oxidationsprodukte aufgrund der außerordentlich hohen Selektivität der erfindungsgemäß verwendeten Gold-Katalysatoren eine sehr hohe Reinheit auf. Die erfindungsgemäß erhaltenen Oxidationsprodukte zeichnen sich gegenüber herkömmlichen Produkten auch dadurch aus, dass sie frei von möglicherweise gesundheitsschädlichen mikrobiellen Stoffwechselprodukten sind.

Besonders handelt es sich bei dem Oxidationsprodukt um Gluconsäure, die durch selektive Oxidation von Glucose erhältlich ist. Die erfindungsgemäß erhaltene Gluconsäure zeichnet sich durch eine hohe Reinheit aus, wobei der Anteil von Gluconsäure am Gesamtoxidationsprodukt mehr als 95%, vorzugsweise mehr als 97%, bevorzugter mehr als 98% und am bevorzugtesten mehr als 99% beträgt.

Unter Verwendung der erfindungsgemäßen Verfahren hergestellte Gluconsäure kann, gegebenenfalls nach weiteren Aufreinigungsschritten unter Verwendung geeigneter Verfahren wie chromatographischer Verfahren, als Zusatz zu Lebensmitteln, Getränken oder Tierfutter, für kosmetische Anwendungen, pharmazeutische Anwendungen oder als Detergens, beispielsweise in Reinigungsmitteln, eingesetzt werden. Gluconsäure besitzt beispielsweise hervorragende Eigenschaften als Antioxidationsmittel. Ferner ist bekannt, dass Gluconsäure ein hervorragendes Mittel zur Hautpflege darstellt.

Beispielsweise handelt es sich bei dem Oxidationsprodukt um Maltobionsäure, die durch selektive Oxidation von Maltose erhältlich ist. Diese Maltobionsäure zeichnet sich durch eine hohe Reinheit aus, wobei der Anteil von Maltobionsäure am Gesamtoxidationsprodukt mehr als 95%, mehr als 97%, mehr als 98% oder mehr als 99% beträgt.

Unter Verwendung der erfindungsgemäßen Verfahren hergestellte Maltobionsäure kann, gegebenenfalls nach weiteren Aufreinigungsschritten, als Zusatz zu Lebensmitteln, Getränken oder Tierfutter oder für pharmazeutische Anwendungen eingesetzt werden. Maltobionsäure kann beispielsweise nach Aufreinigung mittels Ionenaustausch-Chromatographie in die Lacton-Form überführt und dann beispielsweise mit 1,3-Diamino-2-propanal kondensiert werden, wobei das Bisamid von Maltobionsäure erhalten wird. Das Bisamid von Maltobionsäure kann als oral applizierbares Antikoagulant und/oder als Antithrombolytikum eingesetzt werden.

Beispielsweise handelt es sich bei dem Oxidationsprodukt um Lactobionsäure, die durch Oxidation von Lactose erhältlich ist. Diese Lactonbionsäure zeichnet sich durch eine hohe Reinheit aus, wobei der Anteil von Lactobionsäure am Gesamtoxidationsprodukt mehr als 95%, mehr als 97, mehr als 98% oder mehr als 99% beträgt.

Die unter Verwendung der erfindungsgemäßen Verfahren hergestellte Lactobionsäure kann, gegebenenfalls nach weiteren Aufreinigungsschritten, als Zusatz zu Lebensmitteln, Getränken oder Tierfutter und für kosmetische Anwendungen sowie pharmazeutische Anwendungen eingesetzt werden. Aufgrund ihrer hygroskopischen Eigenschaften kann Lactobionsäure atmosphärisches Wasser binden, so dass Lactobionsäure eine natürliche Gelmatrix bildet. Die so gebildete Gelmatrix enthält etwa 14% Wasser. Aufgrund ihrer Fähigkeit, eine solche Gelmatrix zu bilden, stellt Lactobionsäure wie Gluconsäure ein hervorragendes Mittel zur Hautpflege dar. Lactobionsäure weist ferner hervorragende Eigenschaften zur Metallchelat-Bildung auf und lässt sich daher zur Herstellung einer Transplantationslösung, beispielsweise der Wisconsin-Transplantationslösung, einsetzen, die zum Transport und zur Aufbewahrung von zu transplantierenden Organen dient. Lactobionsäure lässt sich auch als Cobuilder in Waschpulver einsetzen, wobei das Waschpulver bis zu 40% Lactobionsäure enthalten kann. Da Lactobionsäure einen milden süß-sauren Geschmack aufweist, kann sie ebenfalls zur Herstellung von Lebensmitteln und Tierfutter eingesetzt werden.

Beispielsweise handelt es sich bei dem Oxidationsprodukt um ein Produktgemisch, das einen hohen Anteil von Gluconsäure und Maltobionsäure enthält und durch Oxidation von Maltodextrin erhältlich ist.

Das durch selektive Maltodextrin-Oxidation erhaltene Maltobionsäure- und Gluconsäure-haltige Oxidationsprodukt kann, gegebenenfalls nach weiteren Aufreinigungsschritten, insbesondere zur weiteren Anreicherung von Maltobionsäure und Gluconsäure, insbesondere zur Herstellung von Lebensmitteln und pharmazeutischen Zusammensetzungen eingesetzt werden.

Beispielsweise handelt es sich bei dem erfindungsgemäß erhaltenen Oxidationsprodukt um ein Produkt, dass einen hohen Anteil an Maltobionsäure aufweist und durch selektive Oxidation eines Stärkesirups erhältlich ist. Das bei der Oxidation von Stärkesirup erhaltene Oxidationsprodukt kann insbesondere zur Herstellung von Lebensmitteln und Tierfutter eingesetzt werden.

Die vorliegende Erfindung betrifft auch die Verwendung eines Kohlenstoff-geträgerten Gold-Katalysators zur C1-selektiven Oxidation eines Oligosaccharids, eines Oligosaccharid-Gemisches oder einer diese(s) enthaltenden Zusammensetzung, wobei das/die Oligosaccharid(e) zu der/den entsprechenden Oligosaccharid-Aldonsäure(n) oxidiert wird. Der erfindungsgemäß verwendete Kohlenstoff-geträgerte Gold-Katalysator enthält etwa 0,1 % bis 5 % Gold, vorzugsweise etwa 0,5 % bis 1% Gold, besonders bevorzugt 0,5 % Gold. Als zu oxidierendes Oligosaccharid kann sowohl eine Oligosaccharid-Aldose als auch ein Oligosaccharid in 2-Ketose-Form eingesetzt werden, wobei das Oligosaccharid in 2-Ketose-Form zunächst in die tautomere Oligosaccharid-Aldose-Form überführt und dann oxidiert wird.

Die vorliegende Erfindung betrifft daher auch die Verwendung eines Kohlenstoff-geträgerten Gold-Katalysators zur Herstellung einer oder mehrerer Oligosaccharid-Aldonsäure(n) aus einer oder mehreren Oligosaccharid-Aldose(n). Die vorliegende Erfindung betrifft auch die Verwendung eines Kohlenstoff-geträgerten Gold-Katalysators zur Herstellung einer oder mehrerer Oligosaccharid-Aldonsäure(n) aus einem oder mehreren Oligosaccharid(en) in 2-Ketose-Form, wobei das/die Oligosaccharid(e) in 2-Ketose-Form vor der Oxidation zunächst in die tautomere(n) Aldose-Form(en) überführt und dann selektiv oxidiert wird/werden.

Beispielsweise ist das unter Verwendung des Kohlenstoff-geträgerten Gold-Katalysators zu oxidierende Oligosaccharid eine Oligosaccharid-Aldose.

Beispielsweise ist die unter Verwendung des Kohlenstoff-geträgerten Gold-Katalysators zu oxidierende Oligosaccharid-Aldose Maltose, Lactose, Cellobiose oder Isomaltose.

Beispielsweise wird unter Verwendung des Kohlenstoff-geträgerten Gold-Katalysators als Maltose-Oxidationsprodukt Maltobionsäure erhalten.

Beispielsweise wird unter Verwendung des Kohlenstoff-geträgerten Gold-Katalysators als Lactose-Oxidationsprodukt Lactobionsäure erhalten.

Beispielsweise liegt das unter Verwendung des Kohlenstoff-geträgerten Gold-Katalysators zu oxidierende Oligosaccharid in 2-Ketose-Form vor, die zunächst in die tautomere Aldose-Form überführt und dann oxidiert wird.

Beispielsweise ist die unter Verwendung des Kohlenstoff-geträgerten Gold-Katalysators zu oxidierende Oligosaccharid-2-Ketose Palatinose.

Beispielsweise ist das unter Verwendung des Kohlenstoff-geträgerten Gold-Katalysators zu oxidierende Kohlenhydrat Maltodextrin.

Beispielsweise ist das unter Verwendung des Kohlenstoff-geträgerten Gold-Katalysators zu oxidierende Kohlenhydrat ein Stärkesirup.

Ein Verfahren zur selektiven Oxidation wird anhand der folgenden Figuren und nicht erfindungsgemäßen Beispiele näher erläutert. Die Figuren zeigen:
Figur 1 zeigt mittels eines UV-Detektors erhaltene Chromatogramme der Produktgemische, die bei der katalytischen Oxidation von Glucose unter Verwendung von Platin- und Palladium-Katalysatoren erhalten wurden. A: Pt-Bi/C-Katalysator; B: Pt/Al₂O₃-Katalysator.
Figur 2 zeigt mittels eines UV-Detektors erhaltene Chromatogramme der Produkte, die erfindungsgemäß bei der katalytischen Oxidation von Glucose unter Verwendung von Gold-Katalysatoren erhalten wurden. A: 0,95% Au/Al₂O₃-Katalysator; B: 0,5% Au/TiO₂-Katalysator.
Figur 3 zeigt mittels eines UV-Detektors erhaltene Chromatogramme der Produkte, die bei der katalytischen Oxidation von Lactose unter Verwendung von Platin-, Palladium- und Gold-Katalysatoren erhalten wurden. A: Pt/Al₂O₃-Katalysator; B: Pd/Al₂O₃-Katalysator; C: 0,5% Au/TiO₂-Katalysator.
Figur 4 zeigt mittels eines UV-Detektors erhaltene Chromatogramme der Produkte, die bei der katalytischen Oxidation von Maltose unter Verwendung von Platin-, Palladium- und Gold-Katalysatoren erhalten wurden. A: Pt/Al₂O₃-Katalysator; B: Pd/Al₂O₃-Katalysator; C: 0,5% Au/TiO₂-Katalysator.
Figur 5 zeigt mittels eines UV-Detektors erhaltene Chromatogramme der Produkte, die bei der katalytischen Oxidation von Maltodextrin unter Verwendung von Platin-, Palladium- und Gold-Katalysatoren erhalten wurden. A: Pt/Al₂O₃-Katalysator; B: Pd/Al₂O₃-Katalysator; C: 0,5% Au/TiO₂-Katalysator.

### Beispiel 1

### Glucose-Oxidation mit Platin- und Palladium-Katalysatoren

Als Katalysatoren wurden ein 5 % Platin- und 5 % Bismut-enthaltender Kohlenstoff-geträgerter Katalysator (Fa. Degussa), ein 5 % Platin-enthaltender Katalysator auf einem Al₂O₃-Träger (Fa. Engelhard) sowie ein 5 % Palladium-enthaltender Katalysator auf einem Al₂O₃-Träger eingesetzt. Die Glucoseoxidation erfolgte unter folgenden Reaktionsbedingungen:

| | |
|---|---|
| Reaktionsvolumen (Batch): | 500 ml |
| Katalysatormenge: | 1 g/l |
| Substratanfangskonzentration: | 100 mmol/l |
| pH-Wert: | 11 |
| Temperatur: | 40°C |
| Druck: | 1 bar |
| O₂-Begasungsrate: | 500 ml/min |
| Rührgeschwindigkeit: | 700 UpM |

Die Ergebnisse, die unter Verwendung der vorstehend aufgeführten Katalysatoren bei der Glucoseoxidation erhalten wurden, sind in der folgenden Tabelle 1 dargestellt.

**Tabelle 1:**

| Vergleich der Pt- und Pd-Katalysatoren bezüglich Glucose-Oxidation, Anfangsaktivität bis 10 % Umsatz und Bildung von Fructose durch Isomerisierung | | | |
|---|---|---|---|
| **Eigenschaft** | **Pt(5%)** | **5 %** | **5 %** |
| | **Bi(5%)/C** | **Pt/Al₂O₃** | **Pd/Al₂O₃** |
| Glucoseumsatz in %* | >95 | >95 | 90 |
| Anfangsaktivität in mmol_{Gluco- se}/ (gₘₑₜₐₗₗ · min) | 19 | 62 | 76 |
| Selektivität zu Gluconsäure in % | 85 | 83 | 92 |
| Selektivität zu anderen Oxidationsprodukten in % ** | 11 | 12 | 4 |
| Selektivität zu Fructose in % | 2, 5 | 2, 5 | <0,5 |

| | | | |
|---|---|---|---|
| * Umsatz, bei dem die Reaktion abgebrochen wurde ** andere Oxidationsprodukte: Glucuronsäure, 2- und 5-Keto-Gluconsäure sowie Glucarsäure | | | |

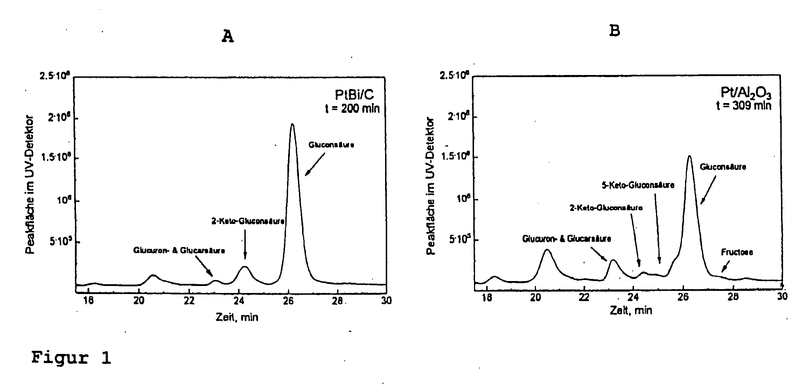

Tabelle 1 zeigt, dass herkömmliche Platin- und Palladium-Katalysatoren bei der Glucose-Oxidation nur eine mäßige Selektivität bezüglich der Bildung von Gluconsäure aufweisen und dass erhebliche Mengen an anderen Oxidationsprodukten wie Glucoronsäure, Glucarsäure, 2-Keto-Gluconsäure und 5-Keto-Gluconsäure gebildet werden. Dieses Produktspektrum ist auch in Figur 1 zu erkennen, die mittels eines UV-Detektors erhaltene Produkt-Chromatogramme zeigt. Aus den erhaltenen Ergebnissen geht hervor, dass die herkömmlicherweise verwendeten Platin- oder Palladium-Katalysatoren für die selektive Herstellung von Gluconsäure durch Glucose-Oxidation nicht geeignet sind.

### Beispiel 2:

### Glucose-Oxidation unter Verwendung von Gold-Katalysatoren

Für dieses Beispiel wurden die folgenden Katalysator-Typen eingesetzt:

| | |
|---|---|
| A: | 1 % Au/C Typ 138 (ACA) |
| B: | 0,95 % Au/Al₂O₃ (ACA) |
| C: | 0,7 % Au/C Typ 11 (ACA) |
| D: | 1 % Au/TiO₂ Typ 5 (ACA) |
| E: | 0,5 % Au/TiO₂ Typ 102 (ACA) |
| F: | 0,5 % Au/TiO₂ Typ 149 (ACA) |

Die Glucose-Oxidation erfolgte unter folgenden Reaktionsbedingungen:

| | |
|---|---|
| Reaktionsvolumen (Batch): | 500 ml |
| Katalysatormenge: | 1 g/l |
| Substratanfangskonzentration: | 100 mmol/l |
| pH-Wert: | 11 |
| Temperatur: | 40°C |
| Druck: | 1 bar |
| O₂-Begasungsrate: | 500 ml/min |
| Rührgeschwindigkeit: | 700 UpM |

Die Ergebnisse, die bei der Glucose-Oxidation unter Verwendung der vorstehend aufgeführten Katalysatoren erhalten wurden, sind in Tabelle 2 dargestellt.

**Tabelle 2:**

| Vergleich verschiedener Gold-Katalysatoren bezüglich Selektivität der Glucose-Oxidation, Anfangsaktivität bis 10 % Umsatz und Fructose-Bildung infolge Isomerisierung | | | | | | |
|---|---|---|---|---|---|---|
| **Eigenschaft** | **A** | **B** | **C** | **D** | **E** | **F** |
| Glucoseumsatz in %* | 93 | 97 | 88 | 74 | 95 | 100 |
| Anfangsaktivität in mmol_{Gluco- se}/g_{Metall} ·min) | 156 | 133 | 390 | 105 | 371 | 550 |
| Selektivität zu Gluconsäure in % | 95 | 95 | 97 | 95 | 98 | 98 |
| Selektivität zu anderen Oxidationsprodukten-Produkten in %** | 0 | <2 | <1 | 0 | 0 | <1 |
| Selektivität zu Fructose in % | 5 | 3 | 2 | 5 | 1 | 1 |

| | | | | | | |
|---|---|---|---|---|---|---|
| * Umsatz, bei dem die Reaktion abgebrochen wurde ** andere Oxidationsprodukte: Glucuronsäure, 2- und 5-Keto-Gluconsäure, Glucarsäure | | | | | | |

Aus Tabelle 2 geht hervor, dass die eingesetzten Gold-Katalysatoren eine Aktivität bezüglich der Glucose-Oxidation aufweisen, die gegenüber der Aktivität der in dem Vergleichsbeispiel verwendeten Platin- und Palladium-Katalysatoren um mindestens eine Größenordnung oder noch mehr höher ist.

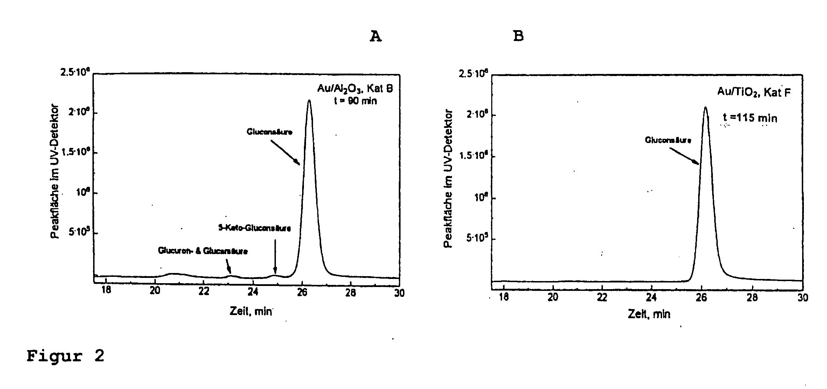

Tabelle 2 zeigt darüber hinaus die außerordentlich hohe Selektivität der verwendeten Gold-Katalysatoren für die Bildung von Gluconsäure aus Glucose. Die hohe Selektivität der eingesetzten Gold-Katalysatoren bezüglich der Gluconsäure-Herstellung ist auch in Figur 2 zu erkennen. Bei Verwendung des Au/TiO₂-Katalysatores (Katalysator F) (Figur 2B) wird nur Gluconsäure gebildet, während andere Oxidationsprodukte nicht nachweisbar sind. Hingegen werden bei Verwendung des Au/Al₂O₃-Katalysators (Katalysator B) neben Gluconsäure auch geringe Mengen anderer Oxidationsprodukten wie Glucuronsäure, Glucarsäure und 5-Keto-Gluconsäure gebildet. Die Menge dieser Oxidationsprodukte ist jedoch im Vergleich zu den Mengen dieser Oxidationsprodukte, die bei Verwendung von Platin- oder Palladium-Katalysatoren erhalten werden, deutlich geringer.

Bei den erfindungsgemäß eingesetzten Gold-Katalysatoren wurde auch festgestellt, dass diese nur zu einer sehr geringen Isomerisierung von Fructose führen, wobei die Fructose selbst aber nicht weiter oxidiert wird.

Anhand der erhaltenen Ergebnisse zeigt sich, dass die erfindungsgemäß eingesetzten Gold-Katalysatoren erheblich besser zur Herstellung von Gluconsäure aus Glucose geeignet sind als die im Vergleichsbeispiel verwendeten Platin- oder Palladium-basierten Katalysatoren.

### Beispiel 3:

### Glucose-Oxidation mit Au/TiO₂-Katalysatoren

Der in Beispiel 2 dargestellte 0,5 % Au-enthaltende Katalysator mit TiO₂-Träger (Katalysator G) wurde für weitergehende Untersuchungen ausgewählt, wobei die Glucose-Oxidation unter variierenden Reaktionsbedingungen getestet wurde.

Sofern nicht anders angegeben, wurden die gleichen Reaktionsbedingungen eingesetzt, wie in Beispiel 2 angeführt.

### Einfluss des pH-Wertes auf die Glucose-Oxidation

Die Ergebnisse der Glucose-Oxidation unter Verwendung des 0,5 % Au/TiO₂-Katalysators (Katalysator G) bei unterschiedlichen pH-Werten sind in Tabelle 3 dargestellt.

**Tabelle 3:**

| Glucose-Oxidation mit einem 0,5% Au//TiO₂-Katalysator bei unterschiedlichen pH-Werten, Anfangsaktivität bis 10% Umsatz, Bildung von Fructose infolge Isomerisierung | | | |
|---|---|---|---|
| **Eigenschaft** | **PH 7** | **pH 9** | **pH 11** |
| Glucoseumsatz in %* | 80 | 100 | 100 |
| Anfangsaktivität in mmol_{Gluco- se}/ (g_{Metall} ·min) | 194 | 416 | 550 |
| Selektivität zu Gluconsäure in % | >99 | >99, 5 | 98 |
| Selektivität zu anderen Ox.-produkten in %** | <0,5 | 0 | <0,5 |
| Selektivität zu Fructose in % | 0 | 0 | 1 |

| | | | |
|---|---|---|---|
| * Umsatz, bei dem die Reaktion abgebrochen wurde ** andere Oxidationsprodukte: Glucuronsäure, 2- und 5-Keto-Gluconsäure, Glucarsäure | | | |

Aus Tabelle 3 geht hervor, dass die Aktivität des Au/TiO₂-Katalysators im nur schwach alkalischen bis neutralen pH-Bereich etwas geringer ist, während die Selektivität noch zunimmt.

### Einfluss der Temperatur auf die Glucose-Oxidation

Die Ergebnisse der Glucose-Oxidation unter Verwendung des Au/TiO₂-Katalysators bei unterschiedlichen Temperaturen, einem pH-Wert von 9 und einer Glucose-Anfangskonzentration von 250 mmol/l sind in Tabelle 4 dargestellt.

**Tabelle 4:**

| Glucose-Oxidation mit einem 0,5% Au//TiO₂-Katalysator bei unterschiedlichen Temperaturen, einer Glucose-Anfangskonzentration von 250 mmol/l und einem pH-Wert von 9, Anfangsaktivität bis 10% Umsatz, Bildung von Fructose infolge Isomerisierung | | | |
|---|---|---|---|
| **Eigenschaft** | **40°C** | **50°C** | **70°C** |
| Glucoseumsatz in %* | 95 | 100 | 100 |
| Anfangsaktivität in mmol_{Glucose}/ (g_{Metall} ·min) | 320 | 1056 | 1404 |
| Selektivität zu Gluconsäure in % | >99,5 | >99 | 95,5 |
| Selektivität zu anderen Ox.-produkten in %** | <0,1 | <0,1 | <0,2 |
| Selektivität zu Fructose in % | 0 | <0,5 | 3 |

| | | | |
|---|---|---|---|
| * Umsatz, bei dem die Reaktion abgebrochen wurde ** andere Oxidationsprodukte: Glucuronsäure, 2- & 5-Keto-Gluconsäure, Glucarsäure | | | |

Aus Tabelle 4 geht hervor, dass die Selektivität der Glucose-Oxidation mit steigender Reaktionstemperatur etwas abnimmt. Dies ist jedoch nicht auf die Bildung anderer Oxidationsprodukte als Gluconsäure zurückzuführen, sondern nahezu ausschließlich auf die vermehrte Bildung von Fructose entsprechend einer höheren Isomerisierungsgeschwindigkeit von Glucose zu Fructose.

### Einfluss der Glucose-Anfangskonzentration auf die Glucose-Oxidation

Der Einfluss der Glucose-Anfangskonzentration auf die Glucose-Oxidation unter Verwendung des 0,5% Au/TiO₂-Katalysators bei einer Temperatur von 40°C und einem pH-Wert von 9 ist in Tabelle 5 dargestellt.

**Tabelle 5:**

| Glucose-Oxidation mit einem 0,5% Au//TiO₂-Katalysator bei unterschiedlichen Glucose-Anfangskonzentrationen und einem pH-Wert von 9, Anfangsaktivität bis 10% Umsatz, Bildung von Fructose infolge Isomerisierung | | | |
|---|---|---|---|
| **Eigenschaft** | **10 mmol/l** | **250 mmol/l** | **1100 mmol/l** |
| Glucoseumsatz in %* | 40 | 95 | 100 |
| Anfangsaktivität in mmol_{Gluco- se}/ (g_{Metall} ·min) | 20 | 320 | 1200 |
| Selektivität zu Gluconsäure in % | 99,4 | 99,6 | 99,2 |
| Selektivität zu anderen Ox.-produkten in %** | 0 | 0 | 0 |
| Selektivität zu Fructose in % | 0, 6 | <0,1 | 0,4 |

| | | | |
|---|---|---|---|
| * Umsatz, bei dem die Reaktion abgebrochen wurde ** andere Oxidationsprodukte: Glucuronsäure, 2- & 5-Keto-Gluconsäure, Glucarsäure | | | |

Aus den in Tabelle 5 dargestellten Ergebnissen ist ersichtlich, dass der verwendete Goldkatalysator auch in konzentrierten Glucose-Lösungen (1100 mmol/l; etwa 20 % Glucose/l) eine ausgezeichnete Aktivität und Selektivität zeigt, wobei deutlich mehr Glucose in gleicher Reaktionszeit umgesetzt wird.

### Beispiel 4:

### Langzeitstabilität des Au/TiO₂-Katalysators bei der Glucoseoxidation

Die Langzeitstabilität des 0,5% Au/TiO₂-Katalysators (Kat G in Beispiel 2) wurde in Form von repeated batch-Versuchen getestet. Dazu wurde tagsüber eine Glucose-Oxidation durchgeführt. Über Nacht beziehungsweise übers Wochenende wurde der Katalysator in der Reaktionslösung, die hauptsächlich aus einer wässrigen Gluconsäure-Lösung besteht, belassen, so dass der Katalysator sedimentieren konnte. Am nächsten Tag wurde der Überstand abdekantiert und der Reaktor wurde mit frischer Glucose-Lösung aufgefüllt und die nächste Glucose-Oxidation wurde durchgeführt. Insgesamt wurden 17 Batch-Versuche bei einem pH-Wert von 9, einer Temperatur von 40°C und einer Glucose-Anfangskonzentration von 250 mmol/l (entspricht etwa 4,5 % Glucose/l) durchgeführt. Die weiteren Reaktionsbedingungen waren wie in Beispiel 2 beschrieben. Die Ergebnisse für einige ausgewählte Batch-Versuche sind in Tabelle 6 aufgeführt.

**Tabelle 6:**

| Langzeitversuch zur Glucose-Oxidation mit einem 0,5% Au//TiO₂-Katalysator bei einer Glucose-Anfangskonzentration von 250 mmol/l, einer Temperatur von 40°C und einem pH-Wert von 9, Anfangsaktivität bis 10% Umsatz, Bildung von Fructose infolge Isomerisierung | | | | | |
|---|---|---|---|---|---|
| **Eigenschaft** | **Batch-Nummer** | | | | |
| | **1** | **5** | **10** | **15** | **17** |
| Glucoseumsatz in %* | 95 | 97 | 99 | 94 | 56 |
| Anfangsaktivität in mmol_{Gluco- se}/ (g_{Metall} ·min) | 320 | 350 | 460 | 430 | 570 |
| Selektivität zu Gluconsäure in % | 99,5 | 99,7 | 99, 6 | 99 | 99,3 |
| Selektivität zu anderen Ox.-produkten in %** | <0,1 | <0,1 | <0,1 | <0,1 | <0,1 |
| Selektivität zu Fructose in % | <0,1 | <0,1 | 0,1 | 0,3 | <0,3 |

| | | | | | |
|---|---|---|---|---|---|
| * Umsatz, bei dem die Reaktion abgebrochen wurde ** andere Oxidationsprodukte: Glucuronsäure, 2- & 5-Keto-Gluconsäure, Glucarsäure | | | | | |

Die in Tabelle 6 dargestellten Ergebnisse zeigen, dass die katalytischen Eigenschaften des Gold-Katalysators über den untersuchten Versuchszeitraum hinweg als konstant anzusehen sind und somit der Katalysator über eine äußerst hohe Lebensdauer verfügt. Insgesamt wurden in diesen Versuchen mit 0,5 g des 0,5 % Au/TiO₂-Katalysators etwa 360 g Glucose nahezu 100%-ig zu Gluconsäure umgesetzt. Dies entspricht etwa 145 t Glucose/kg Gold. Aufgrund des in dieser Rechnung noch nicht berücksichtigten Katalysatorverlustes, der während der Versuche durch Probenentnahme entsteht, liegt dieser Wert wahrscheinlich noch deutlich höher. Ferner ist hier zu berücksichtigen, dass der Katalysator über den untersuchten Zeitraum hinaus vermutlich noch sehr viel länger stabil ist, wodurch dieser Wert wiederum entsprechend ansteigen kann.

### Beispiel 5:

### Selektive Oxidation von Lactose mit Gold-Katalysatoren

Lactose ist ein 1,4 verknüpftes Disaccharid, das aus einem Glucose- und einem Galactose-Teil besteht. Zur Oxidation von Lactose wurde der 0,5 % Au/TiO₂ (Katalysator G) eingesetzt. Zum Vergleich wurden auch der 5 % Pt/Al₂O₃-Katalysator (Engelhard) und der 5 % Pd/Al₂O₃-Katalysator eingesetzt. Die Oxidation von Lactose erfolgte unter folgenden Reaktionsbedingungen:

| | |
|---|---|
| Reaktionsvolumen (Batch): | 500 ml |
| Katalysatormenge: | 1 g/l |
| Substratanfangskonzentration: | 10 mmol/l |
| pH-Wert: | 8 |
| Temperatur: | 80°C |
| Druck: | 1 bar |
| O₂-Begasungsrate | 500 ml/min |
| Rührergeschwindigkeit: | 700 UpM |

Tabelle 7 zeigt die unter Verwendung der verschiedenen Katalysatoren erhaltenen Ergebnisse der katalytischen Lactose-Oxidation.

**Tabelle 7:**

| Verwendung unterschiedlicher Katalysatoren zur Lactose-Oxidation, Anfangsaktivität bis 10% Umsatz | | | |
|---|---|---|---|
| **Eigenschaft** | **5 % Pt/Al₂O₃** | **5 % Pd/Al₂O₃** | **0,5 % Au/TiO₂** |
| Umsatz nach 20 min in %* | <40 | <60 | >95 |
| Anfangsaktivität in mmol_{Substrat}/ g_{Metall} ·min) * | 7,1 | 16,4 | 143 |

| | | | |
|---|---|---|---|
| * abgeschätzt aus Titrationskurve | | | |

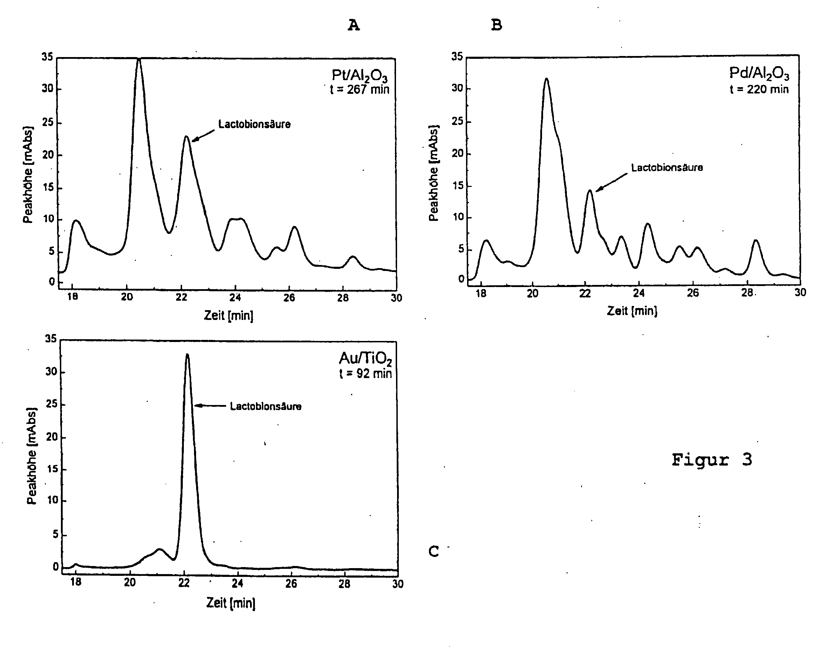

Aufgrund analytischer Probleme kann für die Lactose-Oxidation die Selektivität der einzelnen Katalysatoren nur qualitativ angegeben werden. Dazu lassen sich die im UV-Detektor erhaltenen, in Figur 3 dargestellten Chromatogramme heranziehen, die die oxidierten Reaktionsprodukte zeigen. Aus Figur 3 ist zu ersehen, dass der erfindungsgemäß eingesetzte TiO₂-geträgert Gold-Katalysator eine extrem hohe Selektivität bezüglich der Herstellung von Lactobionsäure besitzt, wobei praktisch keine anderen Oxidationsprodukte nachzuweisen sind. Im Gegensatz dazu führt die Oxidation von Lactose in Gegenwart des Platin- beziehungsweise Palladium-Katalysators zu einem Gemisch unterschiedlicher Produkte, wobei Lactobionsäure nicht das Hauptprodukt darstellt.

### Beispiel 6:

### Selektive Oxidation von Maltose unter Verwendung von Gold-Katalysatoren

Maltose ist ein 1,4-verknüpftes Disaccharid, das aus zwei Glucoseeinheiten besteht. Dabei verfügt ein Glucose-Teil über einen aldehydischen C1-Kohlenstoff. Beide Glucoseeinheiten von Maltose enthalten jeweils ein alkoholisches C6-Kohlenstoffatom. Die Oxidation von Maltose wurde unter Verwendung des 0,5 % Au/TiO₂-Katalysators (Katalysator G) durchgeführt. Zum Vergleich erfolgte die Maltose-Oxidation auch unter Verwendung des 5 % Pt/Al₂O₃-Katalysators (Engelhard) und des 5 % Pd/Al₂O₃-Katalysators. Die Maltose-Oxidation erfolgte unter folgenden Reaktionsbedingungen:

| | |
|---|---|
| Reaktionsvolumen (Batch): | 500 ml |
| Katalysatormenge: | 1 g/l |
| Substratanfangskonzentration: | 10 mmol/l |
| pH-Wert: | 8 |
| Temperatur: | 80°C |
| Druck: | 1 bar |
| O₂-Begasungsrate | 500 ml/min |
| Rührergeschwindigkeit: | 700 UpM |

Tabelle 8 zeigt die bei der Maltose-Oxidation erhaltenen Ergebnisse.

**Tabelle 8:**

| Verwendung unterschiedlicher Katalysatoren zur Maltose-Oxidation, Anfangsaktivität bis 10% Umsatz | | | |
|---|---|---|---|
| **Eigenschaft** | **5 % Pt/Al₂O₃** | **5 % Pd/Al₂O₃** | **0,5 % Au/TiO₂** |
| Umsatz nach 20 min in %* | <80 | 100 | 100 |
| Anfangsaktivität in mmol_{Substrat}/ g_{Metall} ·min) * | 12,3 | 22,2 | 110 |

| | | | |
|---|---|---|---|
| * abgeschätzt aus Titrationskurve | | | |

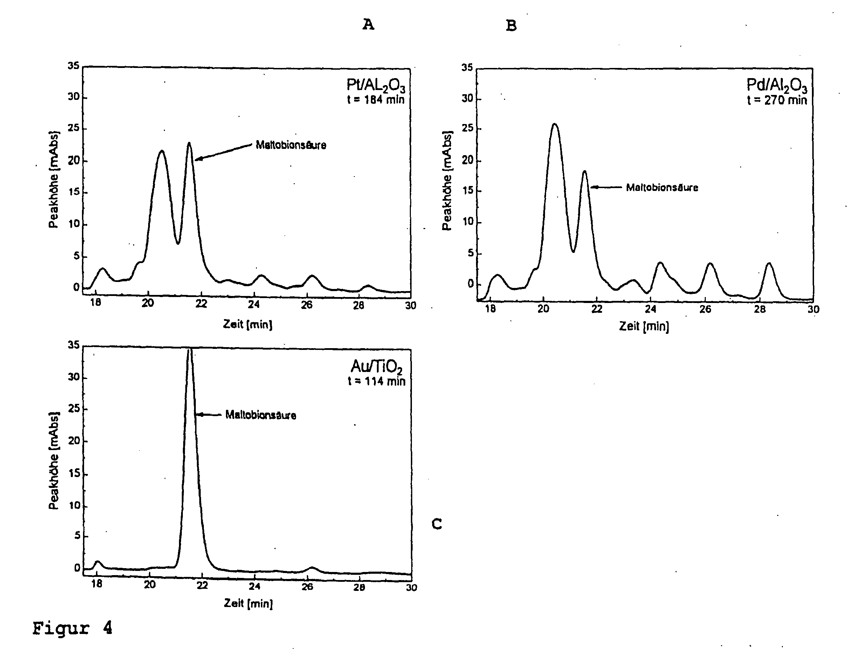

Aufgrund analytischer Probleme kann die Selektivität bezüglich der Oxidation von Maltose zu Maltobionsäure nur qualitativ angegeben werden. Dazu lassen sich die in Figur 4 dargestellten Produkt-Chromatogramme im UV-Detektor heranziehen, anhand derer die oxidierten Reaktionsprodukte nachweisbar sind. Figur 4 zeigt Chromatogramme der bei der Maltose-Oxidation unter Verwendung der angegebenen Katalysatoren erhaltenen Produkte. Aus Figur 4 ist zu ersehen, dass der verwendete Gold-Katalysator im Vergleich zu den Platin- und Palladium-Katalysatoren eine wesentlich höhere Selektivität hinsichtlich der Herstellung von Maltobionsäure besitzt, wobei praktisch keine anderen Oxidationsprodukte nachzuweisen sind. Hingegen werden bei dem Platin- beziehungsweise Palladium-Katalysator auch andere Oxidationsprodukte mit hoher Selektivität gebildet. Diese Ergebnisse zeigen, dass der verwendete Au/TiO₂-Katalysator eine ebenso hohe Selektivität bei der Maltoseoxidation zeigt wie bei der Lactose-Oxidation.

### Beispiel 7:

### Oxidation von Maltodextrin unter Verwendung eines Gold-Katalysators

Maltodextrine stellen Gemische von Oligosacchariden dar, die aus der Verknüpfung von GlucoseEinheiten über eine 1,4-glycosidische Bindung entstehen. Für das Beispiel wurde Agenamalt 20.222 (Maltodextrin DE 19) verwendet. Gemäß Angaben des Herstellers ist das verwendete Maltodextrin DE 19 wie folgt zusammengesetzt:

| | |
|---|---|
| Glucose: | 3,5-4,5 % i.d.TM |
| Maltose: | 3,5-4,5 % i.d.TM |
| Maltotriose: | 4,5-5,5 % i.d.TM |
| Oligosaccharide: | Rest |

Zur Maltodextrin-Oxidation wurden der 5% Pt/Al₂O₃-Katalysator (Engelhard), der 5% Pd/Al₂O₃-Katalysator und der 0,5% Au/TiO₂-Katalysator (Katalysator G) eingesetzt. Die Maltodextrin-Oxidation erfolgte unter folgenden Reaktionsbedingungen:

| | |
|---|---|
| Reaktionsvolumen (Batch): | 500 ml |
| Katalysatormenge: | 1 g/l |
| Substratanfangskonzentration: | 10 mmol/l |
| pH-Wert: | 8 |
| Temperatur: | 80°C |
| Druck: | 1 bar |
| O₂-Begasungsrate | 500 ml/min |
| Rührergeschwindigkeit: | 700 UpM |

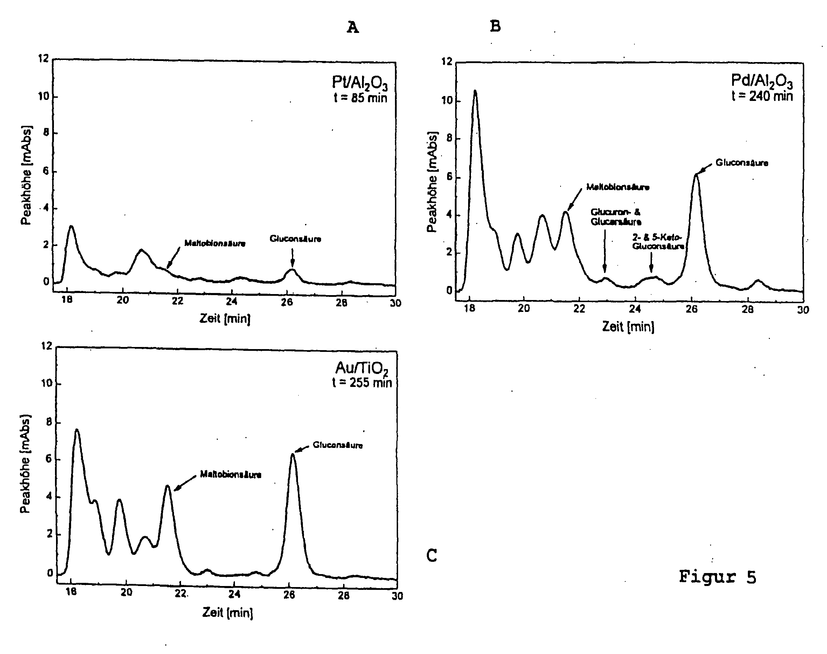

Aufgrund gravierender analytischer Probleme können weder Umsatz noch Aktivität für die einzelnen Katalysator-Typen angegeben werden. Dennoch läuft mit jedem der untersuchten Katalysatoren eine Oxidations-Reaktion ab. Figur 5 zeigt einen qualitativen Vergleich der einzelnen UV-Chromatogramme der Proben bei Reaktionsabbruch.

Aus Figur 5 geht hervor, dass eine beachtliche Oxidation insbesondere mit dem Pd- und dem erfindungsgemäß eingesetzten Au-Katalysator abläuft, was auch durch die Menge an titrierter KOH unterstützt wird (Pt: 0,7 ml; Pd: 2,8 ml; Au: 2,2 ml jeweils bei t = 85 min). Der Vergleich des Produktgemisches, das unter Verwendung des Pd-Katalysators erhalten wurde, mit dem Produktgemisch, das unter Verwendung des Au-Katalysators erhalten wurde, zeigt einen auffälligen Unterschied. Die Substanz mit der Retentionszeit von 20,2 min wird vom Pd-Katalysator mit deutlich höherer Selektivität gebildet als dies beim Au-Katalysator der Fall ist. Die Identität dieser Substanz ist jedoch unbekannt. Hingegen zeigt der erfindungsgemäße Gold-Katalysator eine deutlich höhere Selektivität bezüglich Gluconsäure und Maltobionsäure als der Pd-Katalysator.

## Patentansprüche

1. Verfahren zur selektiven Oxidation von mindestens einem Oligosaccharid, einem Gemisch davon oder einer diese(s) enthaltenden Zusammensetzung, wobei eine wässrige Lösung des Oligosaccharids, des Gemisches oder der Zusammensetzung in Gegenwart eines Gold-Katalysators, umfassend nanodispers verteilte Gold-Partikel auf einem Kohlenstoff-Träger, und von Sauerstoff umgesetzt wird, wobei eine Aldehyd-Gruppe am C1-Kohlenstoffatom des/der Kohlenhydrate(s) selektiv zu einer Carboxyl-Gruppe oxidiert oder eine Aldehyd-Gruppe am C1-Kohlenstoffatom einer 2-Ketose eingeführt und selektiv zu einer Carboxyl-Gruppe oxidiert wird.

2. Verfahren nach Anspruch 1, wobei der Kohlenstoff-geträgerte Gold-Katalysator etwa 0,1 % bis 5 % Gold enthält.

3. Verfahren nach Anspruch 1, wobei der Kohlenstoff-geträgerte Gold-Katalysator etwa 0,5 % bis 1 % Gold enthält.

4. Verfahren nach einem der vorhergehenden Ansprüche, wobei die Oxidation bei einem pH-Wert von 7 bis 11 durchgeführt wird.

5. Verfahren nach einem der vorhergehenden Ansprüche, wobei die Oxidation bei einer Temperatur von 20°C bis 140°C durchgeführt wird.

6. Verfahren nach einem der vorhergehenden Ansprüche, wobei die Oxidation bei einem Druck von 1 bar bis 25 bar durchgeführt wird.

7. Verfahren nach einem der vorhergehenden Ansprüche, wobei das Verhältnis zwischen der Menge des/der zu oxidierenden Oligosaccharide(s) oder des Gemisches davon und der Menge des auf dem Kohlenstoff-Träger enthaltenen Goldes größer als 1000 ist.

8. Verfahren nach einem der vorhergehenden Ansprüche, wobei das zu oxidierende Oligosaccharid eine Disaccharid-Aldose ausgewählt aus der Gruppe, bestehend aus Maltose, Lactose, Cellobiose und Isomaltose ist.

9. Verfahren nach einem der vorhergehenden Ansprüche, wobei die Goldpartikel einen Durchmesser von weniger als 5 nm aufweisen.

10. Verwendung eines Gold-Katalysators, umfassend nanodispers werteilte Gold-Partikel auf einem Kohlenstoff-Trägger, zur C1-selektiven Oxidation von einem Oligosaccharide, einem Oligosaccharid-Gemisch oder einer diese(s) enthaltenden Zusammensetzung in einem in Anspruch 1 charakterisierten Oxidationsverfahren.

11. Verwendung nach Anspruch 10, wobei der Kohlenstoff-geträgerte Gold-Katalysator etwa 0,1 % bis 5 % Gold enthält.

12. Verwendung nach Anspruch 10 oder 11, wobei das zu oxidierende Oligosaccharid eine Oligosaccharid-Aldose ist, ausgewählt aus der Gruppe, bestehend aus Maltose, Lactose, Cellobiose und Isomaltose.

## Claims

1. A method for selective oxidation of at least one oligosaccharide, a mixture thereof or a composition containing these or either of these, wherein an aqueous solution of the oligosaccharide, of the mixture or of the composition is reacted in the presence of a gold catalyst, which comprises nanodispersed gold particles on a carbon support, and oxygen, wherein an aldehyde group on the C1 carbon atom of the carbohydrate(s) is selectively oxidized to form a carboxyl group or an aldehyde group on the C1 carbon atom of a 2-ketose is introduced and selectively oxidized to form a carboxyl group.

2. Method according to claim 1, wherein the carbon-supported gold catalyst contains approximately 0.1 % to 5 % gold.

3. Method according to claim 1, wherein the carbon-supported gold catalyst contains approximately 0.5 % to 1 % gold.

4. Method according to any one of the preceding claims, wherein oxidation is carried out at a pH from 7 to 11.

5. Method according to any one of the preceding claims, wherein oxidation is carried out at a temperature from 20°C to 140°C.

6. Method according to any one of the preceding claims, wherein oxidation is carried out at a pressure from 1 bar to 25 bar.

7. Method according to any one of the preceding claims, wherein the ratio of the amount of oligosaccharide(s) or mixture thereof to be oxidized and the amount of gold contained on the carbon support is larger than 1,000.

8. Method according to any one of the preceding claims, wherein the oligosaccharide to be oxidized is a disaccharide-aldose selected from the group consisting of maltose, lactose, cellobiose and isomaltose.

9. Method according to any one of the preceding claims, wherein the gold particles have a diameter of less than 5 nm.

10. Use of a gold catalyst comprising nanodispersed gold particles on a carbon support for C1-selective oxidation of an oligosaccharide, an oligosaccharide-mixture or a composition containing these or either of these, in an oxidation method as **characterized in** claim 1.

11. Use according to claim 10, wherein the carbon-supported gold catalyst contains approximately 0.1 % to 5 % gold.

12. Use according to claim 10 or 11, wherein the oligosaccharide to be oxidized is an oligosaccharide-aldose selected from the group consisting of maltose, lactose, cellobiose and isomaltose.

## Revendications

1. Procédé pour l'oxydation sélective d'au moins un oligosaccharide, un mélange de celui-ci ou d'une composition contenant celui-ci ou ceux-ci, dans lequel une solution aqueuse du oligosaccharide, du mélange ou de la composition est transformée en présence d'un catalyseur d'or comprenant des particules d'or réparties de manière nanodispersée sur un support en carbone, et d'oxygène, dans lequel un groupe aldéhyde sur l'atome de carbone C1 du/des glucide(s) est oxydé de manière sélective en un groupe carboxyle ou un groupe aldéhyde sur l'atome de carbone C1 d'un 2-cétose est introduit et oxydé de manière sélective en un groupe carboxyle.

2. Procédé selon la revendication 1, dans lequel le catalyseur d'or à support en carbone contient environ 0,1 % à 5 % d'or.

3. Procédé selon la revendication 1, dans lequel le catalyseur d'or à support en carbone contient environ 0,5 % à 1 % d'or.

4. Procédé selon l'une quelconque des revendications précédentes, dans lequel l'oxydation est réalisée à un pH de 7 à 11.

5. Procédé selon l'une quelconque des revendications précédentes, dans lequel l'oxydation est réalisée à une température de 20°C à 140°C.

6. Procédé selon l'une quelconque des revendications précédentes, dans lequel l'oxydation est réalisée à une pression de 1 bar à 25 bars.

7. Procédé selon l'une quelconque des revendications précédentes, dans lequel le rapport entre la quantité du/des oligosaccharide(s) à oxyder ou du mélange de celui-ci et la quantité de l'or contenu sur le support en carbone est supérieur à 1000.

8. Procédé selon l'une quelconque des revendications précédentes, dans lequel l'oligosaccharide à oxyder est un disaccharide de l'aldose choisi dans le groupe constitué par le maltose, le lactose, le cellobiose et l'isomaltose.

9. Procédé selon l'une quelconque des revendications précédentes, dans lequel les particules d'or ont un diamètre inférieur à 5 nm.

10. Utilisation d'un catalyseur d'or comprenant des particules d'or réparties de manière nanodispersée sur un support en carbone pour l'oxydation sélective C1 d'un oligosaccharide, un mélange d'oligosaccharide ou une composition contenant celui-ci ou ceux-ci dans un procédé d'oxydation caractérisé dans la revendication 1.

11. Utilisation selon la revendication 10, dans laquelle le catalyseur d'or à support en carbone contient environ 0,1 % à 5 % d'or.

12. Utilisation selon la revendication 10 ou 11, dans laquelle l'oligosaccharide à oxyder est un oligosaccharide de l'aldose choisi dans le groupe constitué par le maltose, le lactose, le cellobiose et l'isomaltose.
